# EUROPEAN PATENT APPLICATION

(11) **EP 2 604 188 A1**
(43) Date of publication of application: **19.06.2013**
(21) Application number: 12194561.2
(22) Date of filing: 28.11.2012
(51) Int. Cl.: A61B 6/00, G01T 1/24, H01L 27/146, A61B 6/04

(54) **Digital detector and radiation apparatus using the same**

(30) Priority: 15.12.2011 KR 20110135520
(71) Applicant: Samsung Electronics Co., Ltd, Gyeonggi-do 443-742 (KR)
(72) Inventor: Koh, Byoung Hoon, Gyeonggi-do (KR)
(74) Representative: Beck, Michaël Andries T.

(57) **Abstract**

Disclosed herein are a digital detector in which a detection array which detects radiation and readout elements are provided in separate respective housings, and a radiation apparatus which uses the same. The digital detector includes a first housing which includes a detection array and at least one second housing which includes at least one readout element, and the at least one second housing is mounted on the first housing.

## Description

### BACKGROUND

### 1. Field

Exemplary embodiments described herein relate to a digital detector which generates an X-ray radiographic image in a mammographic apparatus.

### 2. Description of the Related Art

In general, a mammographic apparatus is an X-ray photographing apparatus which is used to diagnose diseases of the breast, including breast cancer, at an early stage. The mammographic apparatus irradiates X-rays onto the chest of a patient, and acquires an X-ray radiographic image by detecting the amount of X-rays which have passed through the chest.

Conventionally, an analog image is acquired by detecting the amount of the X-rays which have passed through the chest by using a photosensitive film. However, as digital technology is developed, a digital detector which confirms an image in real time without requiring traditional photographic development and which has properties of excellent data retention and ease in handling has become increasingly widely used. Because the mammographic apparatus is widely used now, development of a digital detector which is applicable to the mammographic apparatus is required.

### SUMMARY

Therefore, it is an aspect of one or more exemplary embodiments to provide a digital detector in which a detection array which detect radiation and one or more readout elements are provided in separate housings.

Additional aspects of the exemplary embodiments will be set forth in part in the description which follows and, in part, will be obvious from the description, or may be learned by practice of the exemplary embodiments.

In accordance with one aspect of an exemplary embodiment, a digital detector includes a first housing which includes a detection array and at least one second housing which includes at least one readout element, wherein the at least one second housing is mounted on the first housing.

The at least one second housing may be mounted on at least one side surface of the first housing.

The at least one second housing may be rotatably mounted on at least one side surface of the first housing.

The first housing may further include a part of the at least one readout element.

The detection array may detect an amount of irradiated radiation and may generate a signal based on the detected amount of irradiated radiation.

The at least one readout element may convert a signal which is generated by the detection array into a digital image signal.

The first housing may further include a scintillator, and, when radiation is irradiated, the scintillator may emit visible rays toward the detection array based on the irradiation of the radiation.

The detection array may be optically connected to the scintillator, and the detection array may include at least one optical sensor which generates a signal which corresponds to an intensity of the rays emitted by the scintillator.

The first housing may further include a photoconductor, and, when radiation is irradiated, the photoconductor may transmit an electrical signal to the detection array based on the irradiation of the radiation.

The detection array may be electrically connected to the photoconductor, and the detection array may generate a signal which corresponds to the transmitted electrical signal.

The detection array may be electrically connected to the at least one readout element.

The digital detector may further include a third housing which includes a power supply which supplies power to each of the detection array and the at least one readout element, and the third housing may be electrically connected to each of the first housing and the at least one second housing.

In accordance with another aspect of an exemplary embodiment, a radiation apparatus includes a radiation irradiator, a digital detector which includes a first housing which includes a detection array and at least one second housing which includes at least one readout element, the at least one second housing being mounted on the first housing, a support structure on which a subject is placed and in which the digital detector is mounted, and a workstation which receives a digital image signal which is generated by the digital detector and which generates an image of an inside portion of the subject. The at least one second housing may be mounted on at least one side surface of the first housing.

The at least one second housing may be rotatably mounted on at least one side surface of the first housing.

The first housing may further include a part of the at least one readout element.

The support structure may include an automatic exposure controller (AEC) which is installed therein.

Respective sizes of each of the first housing and the at least one second housing may be determined such that the first housing is located at an inside portion of the support structure and the at least one second housing is located at an outside portion of the support structure when the digital detector is inserted into the support structure.

In accordance with a further aspect of an exemplary embodiment, a digital detector includes a detection array, a housing which includes the detection array, and a plurality of readout elements which are respectively installed in respective regions of the housing which exclude a region which is occupied by the detection array.

The region which is occupied by the detection array may be located at an inside portion of a support structure when the digital detector is inserted into the support structure, and the respective regions in which each respective one of the plurality of readout elements is installed may be located at an outside portion of the support structure when the digital detector is inserted into the support structure.

### BRIEF DESCRIPTION OF THE DRAWINGS

These and/or other aspects will become apparent and more readily appreciated from the following description of exemplary embodiments, taken in conjunction with the accompanying drawings of which:
FIGS. 1 and 2 are views which illustrate a configuration of a mammographic apparatus, in accordance with an exemplary embodiment;
FIGS. 3, 4, and 5 are views which illustrate a structure of a digital detector, in accordance with an exemplary embodiment;
FIGS. 6A, 6B, and 6C are views which illustrate an external appearance of a digital detector, in accordance with an exemplary embodiment;
FIGS. 7A, 7B, 7C, 7D, and 7E are views which illustrate an insertion of the digital detector into a support structure, in accordance with an exemplary embodiment; and
FIGS. 8A, 8B, and 8C are views which illustrate an external appearance of a digital detector, in accordance with another exemplary embodiment.

### DETAILED DESCRIPTION

Reference will now be made in detail to the exemplary embodiments, examples of which are illustrated in the accompanying drawings, wherein like reference numerals refer to like elements throughout.

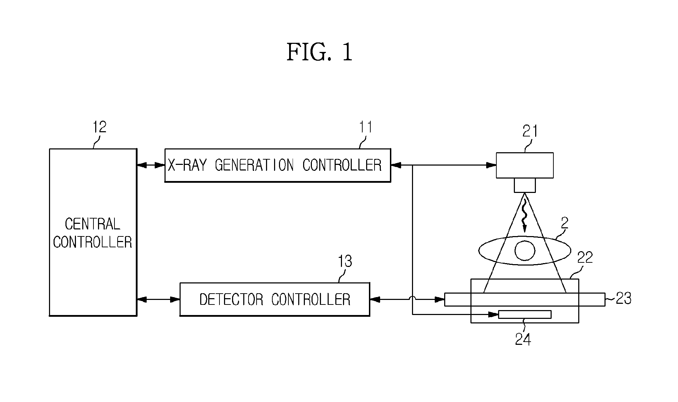

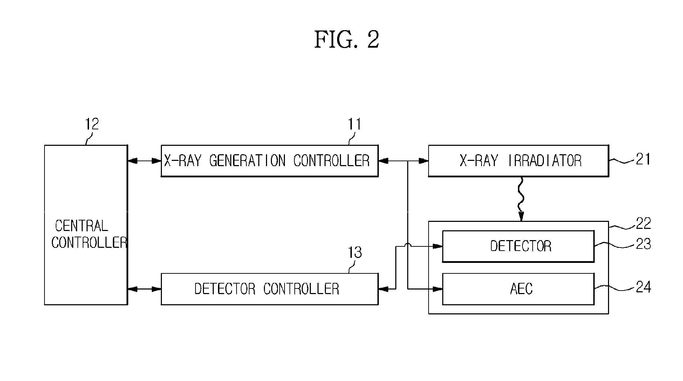

FIGS. 1 and 2 are views which illustrate a configuration of a mammographic apparatus, in accordance with an exemplary embodiment.

As shown in FIGS. 1 and 2, the mammographic apparatus in accordance with an exemplary embodiment includes an X-ray irradiator 21 which irradiates X-rays, a support structure 22 on which a subject, such as, for example, a specific body region 2 of a patient, is located, a digital detector 23 which is mounted in the support structure 22, an automatic exposure controller (AEC) 24 which is mounted in the support structure 22 along a propagation path of X-rays emitted by the X-ray irradiator 21, a digital controller 13 which is connected to the digital detector 23, an X-ray generation controller 11 which is connected to the X-ray irradiator 21 and which is connected to the AEC 24, and a central controller 12 which controls an overall operation of the mammographic apparatus.

The X-ray irradiator 21 generates X-rays, i.e., electromagnetic waves which have a short wavelength and strong penetrating power and which are emitted when electrons which are traveling at a high speed collide with an article. The X-ray irradiator 21 may include a filament which emits thermal electrons and an electrode which forms a strong electric field by using a high voltage.

When a high voltage which is generated by a high voltage power supply is applied to the X-ray irradiator 21, the filament, which acts as a cathode, emits thermal electrons. The emitted thermal electrons propagate in correspondence with the strong electric field and collide with an anode, and X-rays are generated from a local point which has a relatively small size and with which the thermal electrons collide.

The X-ray irradiator 21 may include an iris which adjusts a region toward which the X-rays are irradiated. The iris may be formed by using a material which attenuates X-rays, such as, for example, lead or tungsten.

The support structure 22 supports the body region 2 of the patient which is a target subject of the X-rays which are irradiated from the X-ray irradiator 21.

The digital detector 23, which detects X-rays which have passed through the body region 2 of the patient and then generates a digital image signal based on the detected X-rays, may be inserted into the support structure 22.

Further, the AEC 24, which detects an amount of X-rays which have passed through the digital detector 23 and then transmits a signal to the X-ray generation controller 11 in order to stop the irradiation of X-rays when the detected amount of X-rays exceeds a designated reference value, is mounted in the support structure 22.

The digital controller 13 is electrically connected to the digital detector 23 which is inserted into the support structure 22, controls the digital detector 23, receives a signal generated by the digital detector 23, and transmits the received signal to the central controller 12.

The X-ray generation controller 11 adjusts an amount of X-rays which are irradiated by the X-ray irradiator 21. The X-ray generation controller 11 is connected to the AEC 24, and stops the operation of the X-ray irradiator 21 when the X-ray generation controller 11 receives a signal which indicates that the amount of X-rays has exceeded the designated reference value. Further, the X-ray generation controller 11 may control the operation of the X-ray irradiator 21 in order to determine a region toward which the X-rays are to be irradiated.

The central controller 12 is a microprocessor of a workstation which controls an overall operation of the mammographic apparatus. The central controller 12 executes operations, such as control of the operation of the mammographic apparatus, generation of a radiographic image, and image processing.

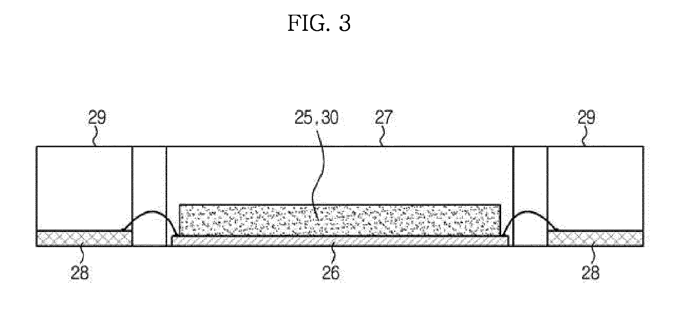

Hereinafter, the structure of the digital detector 23, in accordance with an exemplary embodiment, will be described in detail with reference to FIGS. 3, 4, and 5.

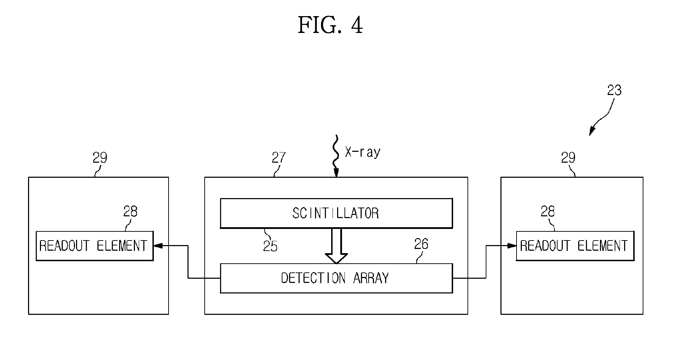

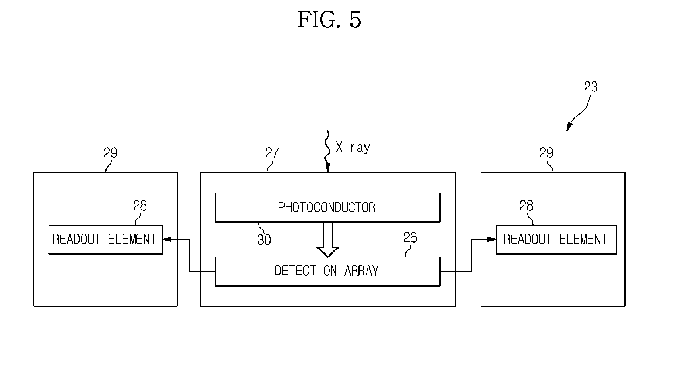

FIG. 3 is a schematic cross-sectional view of the digital detector 23 in accordance with an exemplary embodiment, FIG. 4 is a view which illustrates an indirect conversion type digital detector 23 in accordance with an exemplary embodiment, and FIG. 5 is a view which illustrates a direct conversion type digital detector 23 in accordance with an exemplary embodiment.

As shown in FIG. 3, a first housing 27 includes a detection array 26, and respective second housings 29 include respective readout elements 28. In particular, the detection array 26 and the readout elements 28 are separately provided in the different respective housings 27 and 29.

As shown in FIG. 4, in a detection array 26 of an indirect conversion-type digital detector 23 which changes X-rays to visible rays and then converts the visible rays into an electrical signal by using optical elements, such as optical sensors, pixels, each of which includes an optical sensor and a thin-film transistor (TFT), may be two-dimensionally arranged in a matrix.

The optical sensor may be formed by using a semiconductor material, such as, for example, silicon. In particular, the optical sensor may include a PIN photo diode which has a PIN structure, including a positive (P)-type semiconductor layer, an intrinsic (I)-type semiconductor layer, and a negative (N)-type semiconductor layer, or the optical sensor may include a PN photo diode which has a PN structure, including a positive (P)-type semiconductor layer and a negative (N)-type semiconductor layer.

Further, a scintillator 25 is installed on a front surface of the detection array 26, i.e., a surface of the detection array 26 upon which X-rays are incident. The scintillator 25 may convert X-rays which have passed through the body region 2 of the patient to rays which have a wavelength which is detectable by the detection array 26, for example, visible rays which have a green wavelength.

The scintillator 25 may be formed by using a halogen compound, such as, for example, cesium iodide which is doped with thallium or sodium, or may include an oxide-based compound, such as, for example, gadolinium oxysulfide.

In case of the indirect conversion type digital detector 23, X-rays which are incident upon the digital detector 23 are converted to visible rays by the scintillator 25, and the detection array 26 generates an electrical signal based on an intensity of the visible rays.

As shown in FIG. 5, in a direct conversion-type digital detector 23 which converts X-rays directly into an electrical signal by using a photoconductor 30, the photoconductor 30 is arranged on the front surface of the detection array 26, i.e., a surface of the detection array 26 upon which X-rays are incident.

The photoconductor 30 may be formed, for example, by using one or more of a-Se, CdZnTe, and Hgl2.

When X-rays are incident, the photoconductor 30 generates electron-hole pairs, the generated electron-hole pairs are stored in capacitors, and an electrical signal which results from the electron-hole pairs which are stored in the capacitors is read by the operation of TFTs which are included in the detection array 26. Although FIG. 5 illustrates the photoconductor 30 and the detection array 26 as being provided separately, the direct conversion-type digital detector 23 may include the detection array 26 which includes the photoconductor 30, the capacitors, and the TFTs.

Each of the indirect conversion-type digital detector and the direct conversion-type digital detector 23, in accordance with an exemplary embodiment, includes readout elements 28 which are provided in separate housings and which are used to convert the electrical signal generated by the detection array 26 into a digital image signal.

With reference to FIGS. 3, 4, and 5, the digital detector 23 is constructed by connecting the first housing 27 which includes the detection array 26 with each of the second housings 29 which include the respective readout elements 28.

In an exemplary embodiment, second housings 29 are respectively mounted at both ends of the first housing 27, and thus a total of two second housings 29 are provided. However, the exemplary embodiments are not limited thereto, but at least one second housing 29 may be mounted on the side surface of at least one end of the first housing 27 so as to form the digital detector 23.

If the readout element 28 and the detection array 26 are provided together on one single housing, the readout element 28 is generally arranged on a lower surface of the detection array 26.

Because a circuit composition device, such as the readout element 28, may malfunction if it is exposed to X-rays, the readout element 28 is mounted on a lower surface of the detection array 26, and an X-ray blocking member which is formed by using a material which shields the readout element 28 from X-rays which have passed through the detection array 26 is arranged between the detection array 26 and the readout element 28 in order to protect the readout element 28 from exposure to X-rays.

Because the above-configured detector blocks X-rays which are incident upon the AEC 24 of the mammographic apparatus by action of the X-ray blocking member, such a detector is not used in a state in which the AEC 24 is mounted in the support structure 22.

In order to solve such a problem, when a detector is manufactured, a region of the detector where a body region of a patient will be located is anticipated and accounted for during a design phase, and accordingly, an X-ray blocking member may be positioned on other regions of the detector except for such a region of the detector, and the readout element 28 may be positioned only on the lower surface of the detector at the regions where the X-ray blocking member is positioned. However, if the position of the AEC 24 in the mammographic apparatus corresponds to the regions where the X-ray blocking member is positioned, the above-described problem may remain unresolved.

In the digital detector 23 in accordance with an exemplary embodiment, because the readout element 28 is not positioned on the lower surface of the detection array 26, but is instead separately positioned on each of the second housings 29 which are respectively arranged on both side surfaces of the first housing 27 which includes the detection array 26, i.e., each of the second housings 29 is respectively arranged to be coplanar with the first housing 27, the X-ray blocking member, which is configured to protect the readout element 28 from exposure to X-rays, may be omitted.

Then, the above-described problem does not arise, and thus the AEC 24 of the mammographic apparatus according to an exemplary embodiment may be used without modification.

In an exemplary embodiment, the detection array 26 of the first housing 27 is electrically connected to each of the respective readout elements 28 of the second housings 29, for example, by using wires.

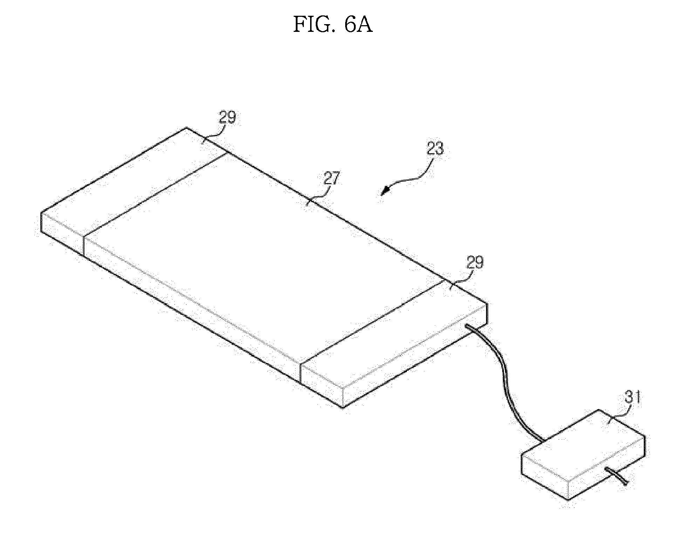

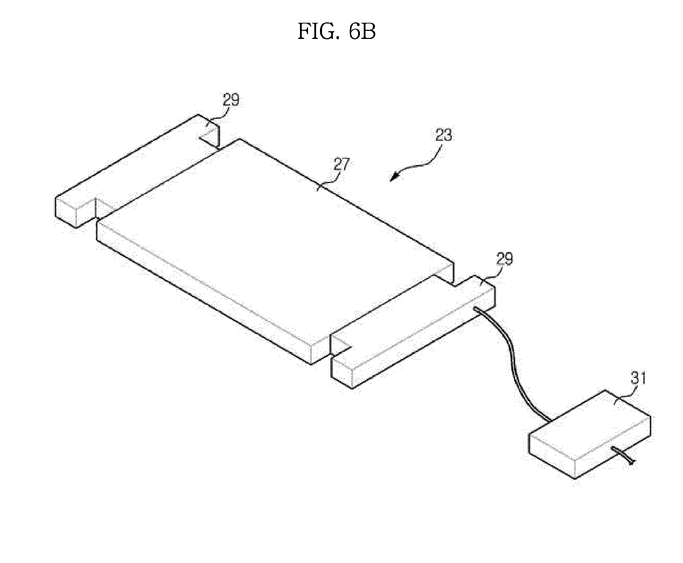

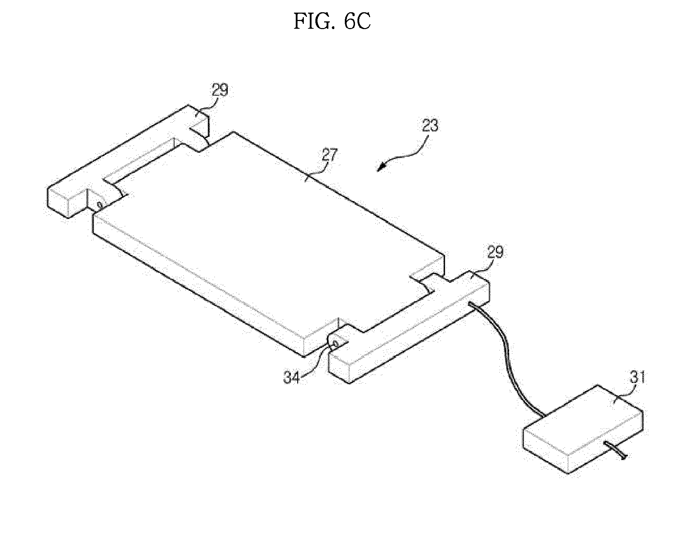

Further, referring to FIG. 6A, FIG. 6B, and FIG. 6C, the digital detector 23 may be electrically connected to a third housing 31 which includes a power supply which supplies power to at least one of the detection array 26 and one or more of the readout elements 28.

Referring to FIG. 6B, recesses having a designated depth may be formed at parts of the second housings 29 which are connected to the first housing 27.

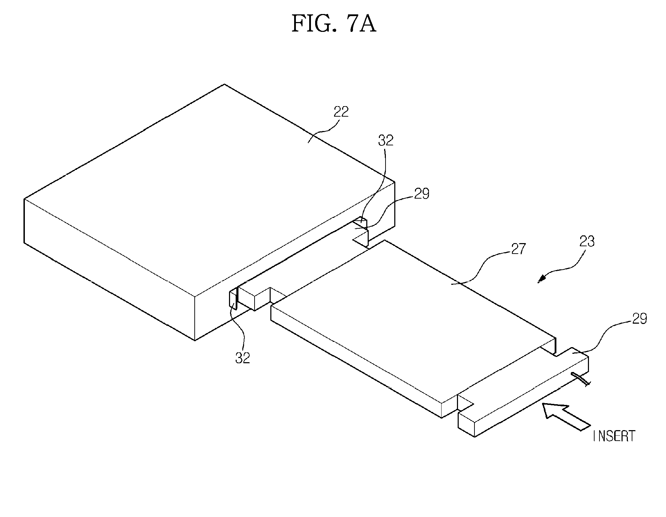

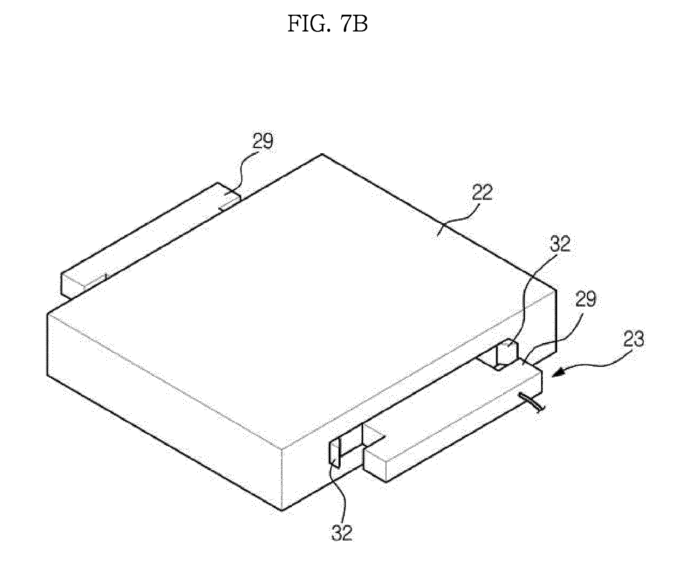

With reference to FIGS. 7A and 7B, when the detector 23 is inserted into the support structure 22, holders 32 which are installed in the support structure 22 are fixed to the recesses so as to inform a user that the detector 23 is properly inserted into the support structure 22.

The sizes of the respective housings of the digital detector 23 may be determined so that the first housing 27 is located at an inside portion of the support structure 22 and the second housings 29 are located at respective outside portions of the support structure 22 when the digital detector 23 is inserted into the support structure 22. Because the second housings 29, which include the respective readout elements 28, are arranged at respective outside portions of the support structure 22 when the digital detector 23 is inserted into the support structure 22, the readout elements 28 are not exposed to X-rays which are irradiated toward the subject 2 which will be placed on the support structure 22.

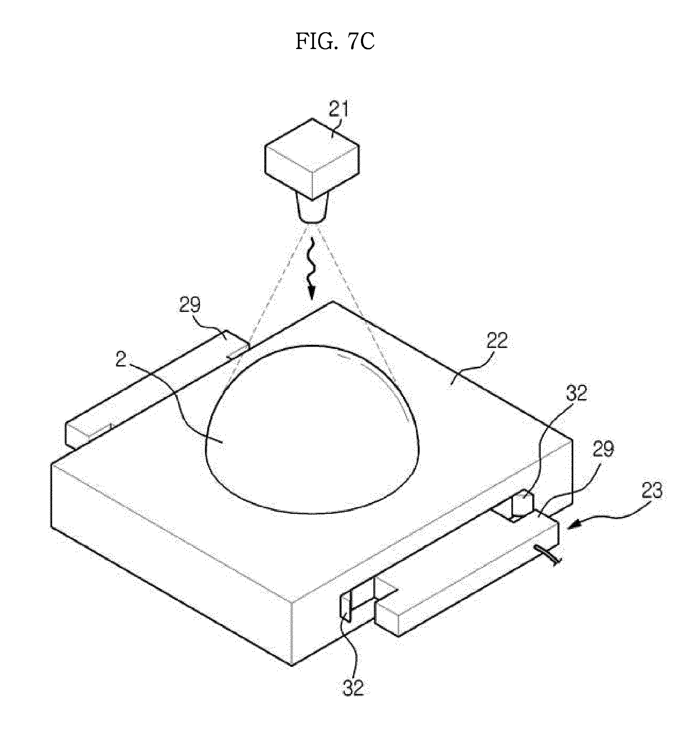

With reference to FIG. 7C, when X-rays are irradiated toward the subject 2, X-rays do not reach the second housings 29.

Further, as shown in FIG. 6C, when the second housings 29 are connected to the first housing 27, each respective second housing 29 may be connected to the first housing 27 such that the respective second housing 29 is rotatable upward and downward about a shaft 34 which connects the second housing 29 to the first housing 27.

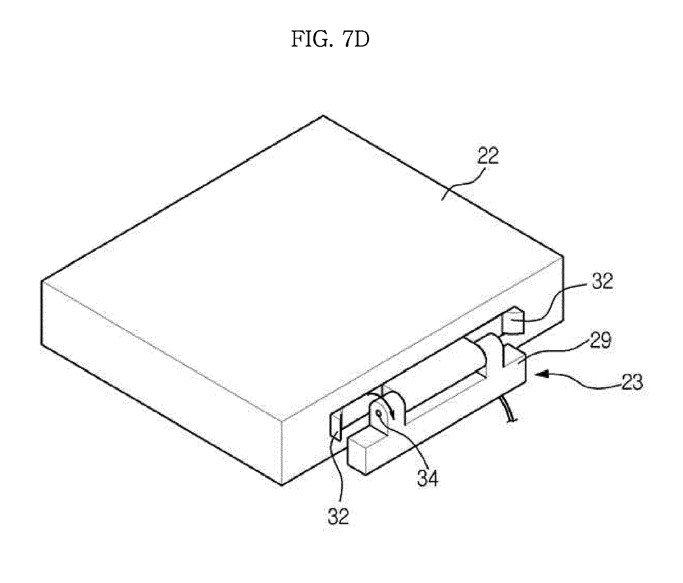

If the respective second housings 29 are connected to the first housing 27 in this way, each of the respective second housings 29 may be folded in the direction toward the lower surface of the support structure 22, as shown in FIG. 7D, after insertion of the digital detector 23 into the support structure 22.

If each of the respective second housings 29 is folded, as shown in FIG. 7D, irradiation of X-rays to each respective second housing 29 is maximally prevented.

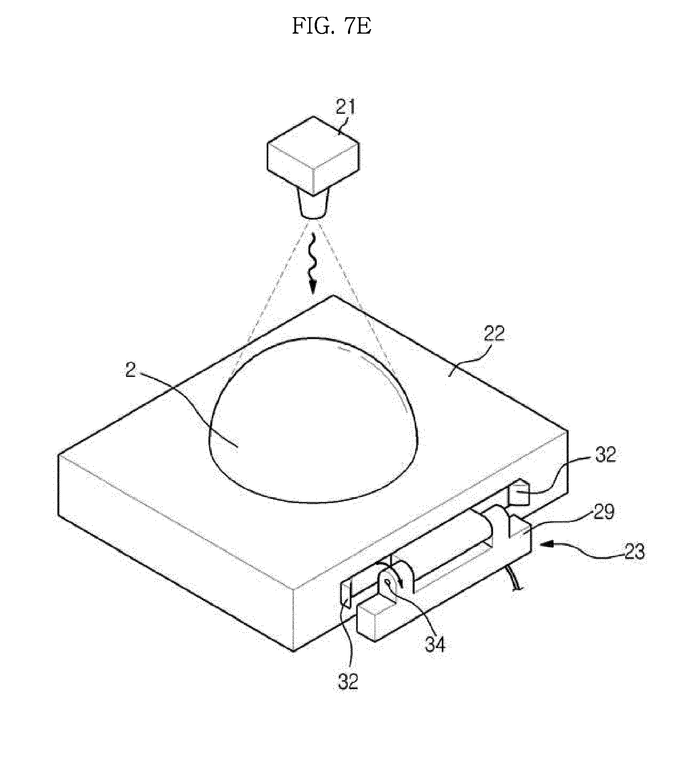

With reference to FIG. 7E, when X-rays are irradiated toward the subject 2, X-rays do not reach the second housings 29. Even if the size of the subject 2 occupies a larger area of the support structure 22 than an area shown in FIG. 7E, the probability of X-rays reaching the second housings 29 is low because the second housings 29 are folded in the direction toward the lower surface of the support structure 22.

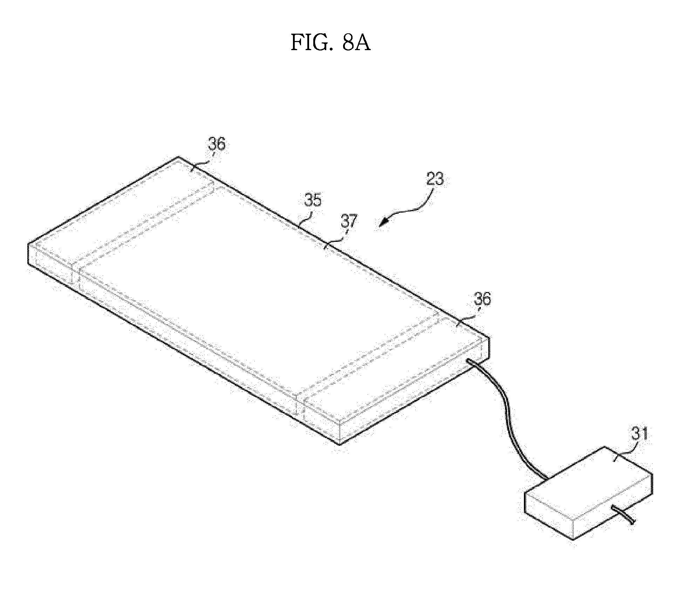

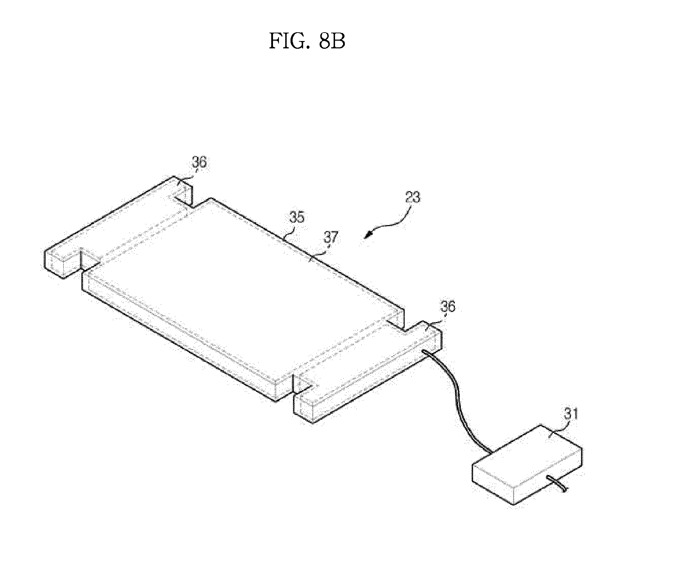

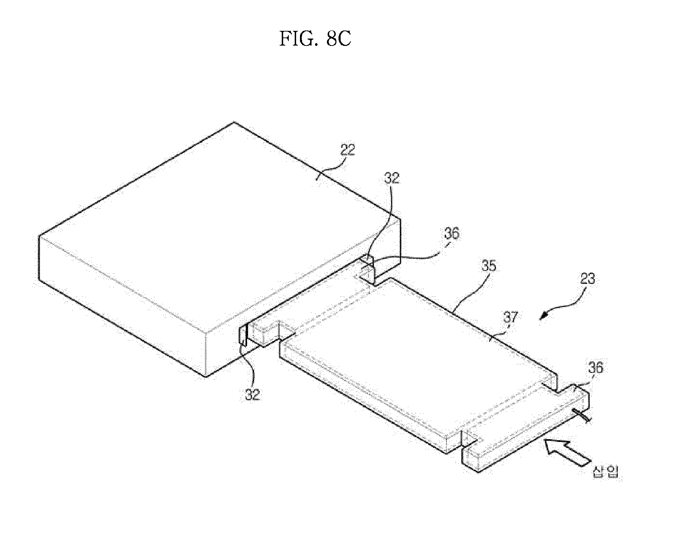

FIGS. 8A and 8B are views which illustrate a digital detector 23 in accordance with another exemplary embodiment, and FIG. 8C is a view which illustrates mounting of the digital detector 23 in a support structure, in accordance with this same exemplary embodiment.

The digital detector 23 in accordance with an exemplary embodiment includes a detection array, readout elements, and a housing 35 which includes the detection array and the readout elements in the same manner as the above-described digital detector 23 in accordance with an exemplary embodiment of the present invention, but differs from the above-described digital detector 23 in that one housing 35 includes all of the detection array and the readout elements.

The readout elements are installed in respective regions 36 of the housing 35 which exclude a region 37 which is occupied by the detection array. In particular, the respective positions of the detection array and the readout elements in the housing 35 may be determined such that the region 37 occupied by the detection array is located at an inside portion of the support structure when the digital detector 32 is inserted into the support structure, and the respective regions 36 in which the readout elements are installed are located at respective outside portions of the support structure when the digital detector 32 is inserted into the support structure.

Recesses may be formed at each of the respective regions 36 of the digital detector 23 in which the readout elements are installed so that holders 32 installed in the support structure may be fixed to the recesses when the digital detector 23 is inserted into the support structure so as to inform a user that the detector 23 is properly inserted into the support structure, as illustrated in FIG. 8C.

As is apparent from the above description, a digital detector in accordance with an exemplary embodiment converts X-rays directly into a digital signal without removing a photosensitive film from a support structure for development, and then transmits the digital signal to a workstation.

Further, respective readout elements are installed in separate respective housings which are mounted on side surfaces of a housing which includes a detection array, thus preventing exposure of the readout elements to X-rays without requiring the use of a separate X-ray blocking member.

Further, because the X-ray blocking member which might otherwise be required in order to prevent the readout elements from being exposed to X-rays if the readout elements and the detection array were installed in the same housing is omitted, an AEC which is used in the conventional mammographic apparatus may be used without modification.

Although a few exemplary embodiments have been shown and described, it will be appreciated by those skilled in the art that changes may be made in these exemplary embodiments without departing from the principles and spirit of the present disclosure, the scope of which is defined in the claims and their equivalents.

## Claims

1. A digital detector comprising:
a first housing which includes a detection array; and
at least one second housing which includes a readout element,
wherein the at least one second housing is mounted on the first housing.

2. The digital detector according to claim 1, wherein the at least one second housing is mounted on at least one side surface of the first housing.

3. The digital detector according to claim 1, wherein the at least one second housing is rotatably mounted on at least one side surface of the first housing.

4. The digital detector according to claim 1, wherein the first housing further includes a part of the readout element.

5. The digital detector according to claim 1, wherein the detection array detects an amount of irradiated radiation and generates a signal according to the detected amount of irradiated radiation.

6. The digital detector according to claim 1, wherein the readout element converts a signal which is generated by the detection array into a digital image signal.

7. The digital detector according to claim 1, wherein:
the first housing further includes a scintillator; and
when radiation is irradiated, the scintillator emits visible rays toward the detection array based on the irradiation of the radiation.

8. The digital detector according to claim 7, wherein:
the detection array is optically connected to the scintillator; and
the detection array includes at least one optical sensor which generates a signal corresponding to the intensity of the rays emitted from the scintillator.

9. The digital detector according to claim 1, wherein:
the first housing further includes a photoconductor; and
when radiation is irradiated, the photoconductor transmits an electrical signal to the detection array based on the irradiation of the radiation.

10. The digital detector according to claim 9, wherein:
the detection array is electrically connected to the photoconductor; and
the detection array generates a signal corresponding to the transmitted electrical signal.

11. The digital detector according to claim 1, wherein the detection array and the readout element are electrically connected.

12. The digital detector according to claim 1, further comprising a third housing which includes a power supply which supplies power to the detection array and the readout element,
wherein the third housing is electrically connected to the first housing and the at least one second housing.

13. A radiation apparatus comprising:
a radiation irradiation unit;
a digital detector which includes a first housing which includes a detection array and at least one second housing which includes a readout element, the at least one second housing being mounted on the first housing;
a support unit on which a subject is placed and in which the digital detector is mounted; and
a workstation which receives a digital image signal generated by the digital detector and which generates an image of an inside of the subject.

14. The radiation apparatus according to claim 13, wherein the support unit includes an automatic exposure controller (AEC) installed therein.

15. The radiation apparatus according to claim 13, wherein respective sizes of each of the first housing and the at least one second housing are determined such that the first housing is located at an inside of the support unit and the at least one second housing is located at an outside of the support unit when the digital detector is inserted into the support unit.
